# EUROPÄISCHE PATENTSCHRIFT

(11) **EP 1 445 307 B1**
(45) Veröffentlichungstag und Bekanntmachung des Hinweises auf die Patenterteilung: **25.07.2007**
(21) Anmeldenummer: 03025298.5
(22) Anmeldetag: 06.11.2003
(51) Int. Cl.: C12M 1/00

(54) **Schüttel-Inkubator**
Agitating incubator
Incubateur à agitation

(30) Priorität: 24.01.2003 DE 10302809
(43) Veröffentlichungstag der Anmeldung: 11.08.2004
(73) Patentinhaber: Thermo Electron LED GmbH, 63505 Langenselbold (DE)
(72) Erfinder: Betz, Stefan, 63526 Erlensee (DE); Heeg, Hubert, 63776 Mömbris (DE); Melching, Achim, 63505 Langenselbold (DE)
(74) Vertreter: Lang, Friedrich

(56) Entgegenhaltungen:
- EP-A- 0 569 214
- US-A1- 2002 063 077

## Beschreibung

Die Erfindung betrifft einen Schüttel-Inkubator mit mindestens einer Objekt-Lagervorrichtung, welche mehrere übereinander angeordnete Objekt-Lagerplätze aufweist.

Unter einer Objekt-Lagervorrichtung wird im vorliegenden Zusammenhang eine Vorrichtung verstanden, in die Objekte an mehreren, in der Art eines Turmes übereinander angeordneten, Objekt-Lagerplätzen aufgenommen werden können. Als Objekte werden im weiteren offene Schalen, geschlossene Behälter, sogenannte Mikrotiterplatten und ähnliche Behältnisse zur Aufnahme von Proben verstanden. Vor allem in der Forschung und der industriellen Fertigung finden diese Objekte vielfach Verwendung.

Grundsätzlich sind Schüttel-Inkubatoren aus der Literatur bekannt. So offenbart die europäische Patentanmeldung EP 0569214 A2 einen Schüttler/Inkubator, der eine gerade Anzahl an turmartig übereinander angeordneten Schüttelebenen umfasst, die mittels eines Excenterantriebes in eine Schüttelbewegung versetzt werden können. Der wesentliche Nachteil dieser Vorrichtung besteht darin, dass alle Schüttelebenen durch einen gemeinsamen Excenterantrieb gleichzeitig bewegt werden. Dadurch ist es unmöglich, einzelne der übereinander angeordneten Schüttelebenen zu bewegen, während andere Schüttelebenen des Turmes ruhen. Auch gestattet der Excenterantrieb lediglich für alle Schüttelebenen des Turmes die gleiche Stärke der Schüttelbewegung festzulegen.

Der Erfindung liegt deshalb die Aufgabe zugrunde, einen Schüttel-Inkubator der eingangs genannten Art anzugeben, welcher es ermöglicht, die in einer Objekt-Lagervorrichtung befindlichen Objekte individuell und voneinander unabhängig zu schütteln.

Diese Aufgabe wird erfindungsgemäß durch die Merkmale des Anspruchs 1 gelöst, wobei zweckmäßige Weiterbildungen der Erfindung durch die Merkmale der Unteransprüche gezeigt werden.

Erfindungsgemäß ist vorgesehen, dass an mehreren Objekt-Lagerplätzen je eine Schütteleinheit angeordnet ist und jede Schüttelplattformindividuell und von den anderen Schüttelplattformen unabhängig von der mindestens einen Steuereinheit ansteuerbar ist. Individuell und voneinander unabhängig bedeutet in diesem Zusammenhang, dass die Schüttelbewegung, also zum Beispiel die Schüttelfrequenz oder die Amplitude (Schüttelhöhe), für jede einzelne Schüttelplattform durch die mindestens eine Steuereinheit festgelegt und gesteuert werden kann.
Dadurch ist es möglich, eine Objekt-Lagervorrichtung mit verschiedenen Objekten zu bestücken und diese den individuellen Erfordernissen hinsichtlich Schüttelbewegung und Schütteldauer zu bewegen. Ist eine Schüttelbewegung für ein Objekt nicht vorgesehen, so fungiert der Objekt-Lagerplatz dieser Schütteleinheit aufgrund der ruhenden Schüttelplattform als statischer Lagerplatz. Dem Fachmann sind zahlreiche Anwendungen bekannt, bei denen ein Objekt unter Inkubationsbedingungen in zeitlicher Abfolge abwechselnd geschüttelt und gelagert werden muss.
Durch die Erfindung wird folglich die Möglichkeit geschaffen, mehrere Objekte durch ein automatisiertes Transportsystem zeitlich versetzt in ein und dieselbe Objekt-Lagervorrichtung einzubringen und die Objekte unter Inkubationsbedingungen nach einem individuellen Programm zu schütteln bzw. zu lagern.

In einer ersten bevorzugten Ausführungsform der Erfindung ist die Grundeinheit der Schütteleinheit fest mit der Objekt-Lagervorrichtung verbunden. Dadurch wird einerseits eine dauerhafte und besonders stabile Befestigung mit der Objekt-Lagervorrichtung gewährleistet, andererseits eine zusätzliche Stabilisierung der Objekt-Lagervorrichtung herbeigeführt.

Eine alternative bevorzugte Weiterbildung sieht hingegen an einem Objekt-Lagerplatz eine lösbare Halterung für die Schütteleinheit einer Objekt-Lagervorrichtung vor, so dass die Schütteleinheit bei Bedarf aus der Objekt-Lagervorrichtung entfernt werden kann. Grundsätzlich kommen alle im Stand der Technik bekannten lösbaren Halterungen in Frage. Beispielsweise kann die Schütteleinheit durch zwei an der Objekt-Lagervorrichtung befestigte Schienen gehaltert und das Verrutschen der Schütteleinheit durch eine lösbare Rastverbindung verhindert werden.

Weiterhin ist vorteilhaft vorgesehen, dass die Objekt-Lagerstelle der Schütteleinheit ausgebildet ist, um durch ein automatisiertes Transportsystem mit einem Objekt bestückt zu werden und um mit einem automatisierten Transportsystem ein Objekt von der Objekt-Lagerstelle zu entfernen.

Dazu ist in einer bevorzugten Ausführungsform vorgesehen, dass die Objekt-Lagerstelle der Schütteleinheit ein auf der Schüttelplattform angeordnetes Distanzelement umfasst, welches Freiraum zur Manipulation eines sich auf der Objekt-Lagerstelle befindlichen Objektes schafft. Das Distanzelement kann grundsätzlich alle dem Fachmann bekannten Formen aufweisen. Geeignet wäre beispielsweise ein einteiliges, U-förmiges Distanzelement oder ein aus zwei Teilen bestehendes Distanzelement. In beiden Fällen wird das Distanzelement so auf der Schüttelplattform angeordnet, dass ein durch eine Transporteinrichtung von unten gehaltertes Objekt problemlos auf die Objekt-Lagerstelle gelegt und die Transporteinrichtung wieder entfernt werden kann.

Zweckmäßig ist es auch, dass die Objekt-Lagerstelle einer Schütteleinheit mindestens ein auf der Schüttelplattform oder dem Distanzelement angeordnetes Spannelement umfasst. So kann das Verrutschen eines auf der Objekt-Lagerstelle befindlichen Objektes wirkungsvoll unterbunden werden. Das Spannelement kann ein- oder mehrteilig sein. Als mehrteiliges Spannelement sind beispielsweise vier auf den Ecken der Objekt-Lagerstelle angeordnete Fixierwinkeln geeignet.

In einer weiteren bevorzugten Ausführungsform ist außerhalb des Inkubator-Arbeitsraumes mindestens eine Steuerung und Stromversorgung der Schütteleinheiten angeordnet, von der aus eine Steuer-/Versorgungsleitung in den Inkubatorarbeitsraum führt, wobei die Steuer-/Versorgungsleitung im Inkubatorarbeitsraum einen Leitungsanschluss aufweist. Die wenigstens eine Steuereinheit umfasst die dem Fachmann bekannten Komponenten, die notwendig sind, um die Stromversorgung sowie die Steuerung der im Inkubatorarbeitsraum befindlichen Schütteleinheiten zu gewährleisten. So kann die Steuereinheit beispielsweise eine Recheneinheit umfassen, mit welcher der individuelle Bewegungsablauf der Schüttelplattformen der Schütteleinheiten festgelegt wird.

Zweckmäßigerweise wird die Schütteleinheit über eine lösbare Leitungsverbindung an den Leitungsanschluss der mindestens einen Steuereinheit angeschlossen. Dem Fachmann sind verschiedene lösbare Leitungsverbindungen bekannt, die hier Verwendung finden können.

In einer weiteren bevorzugten Ausführungsform ist im Inkubatorarbeitsraum eine Verteilungseinheit zum Anschluss mehrerer Schütteleinheiten angeordnet, wobei die Verteilungseinheit über eine lösbare Leitungsverbindung an den Leitungsanschluss der mindestens einen Steuereinheit angeschlossen ist. Die Verteilungseinheit kann fest oder lösbar, beispielsweise an der Inkubatorarbeitsraumwand gehaltert werden. Der Leitungsanschluss der Schütteleinheiten an die Verteilungseinheit kann dem Stand der Technik entsprechend, fest oder lösbar, erfolgen. Ein lösbarer Leitungsanschluss zwischen Schüttel- und Verteilungseinheit wird vor allem dann sinnvoll sein, wenn die Schütteleinheit bei Bedarf aus der Objekt-Lagervorrichtung entfernt werden soll.

Bei einer alternativen bevorzugten Weiterbildung ist vorgesehen, eine Verteilungseinheit an einer Objekt-Lagervorrichtung anzuordnen. Dadurch wird die Leitungslänge von der sich in der Objekt-Lagervorrichtung befindlichen Schütteleinheit zur Verteilungseinheit minimiert und die Möglichkeit geschaffen, eine Objekt-Lagervorrichtung schnell und einfach gegen eine andere Objekt-Lagervorrichtung auszutauschen, die beispielsweise eine andere Anzahl an Schütteleinheiten auf der Objekt-Lagervorrichtung aufweist. Ein Austausch der gesamten Objekt-Lagervorrichtung wird zum Beispiel dann zweckmäßig sein, wenn die Höhe der zu lagernden Objekte die Veränderung der Anzahl der sich auf einer Objekt-Lagervorrichtung befindlichen Objekt-Lagerplätze notwendig macht.

Besonders vorteilhaft kann es aber auch sein, an mehrere Objekt-Lagervorrichtungen eine Verteilungseinheit zum Anschluss mehrerer Schütteleinheiten anzuordnen. Insbesondere beim Vorhandensein zahlreicher Schütteleinheiten auf mehreren Objekt-Lagervorrichtungen wird die Leitungslänge von einer Schütteleinheit zur nächsten Verteilungseinheit möglichst klein gehalten und darüber hinaus der bedienungsfreundliche Austausch einzelner Objekt-Lagervorrichtungen ermöglicht.

Weiterhin ist in einer bevorzugten Weiterbildung die Verwendung einer Schütteleinheit vorgesehen, bei der sich die Schüttelplattform nach (Strom-)Abschaltung selbständig auf eine zentrische Nullstellung positioniert. Dadurch wird ein sich auf der Schüttelplattform befindliches Objekt nach Beendigung des Schüttelvorganges in eine zentrische und horizontale Position gebracht, was Voraussetzung für eine automatisierte Bestückung und Entladung der Objekt-Lagerstelle durch eine Transporteinrichtung ist.

Nachfolgend wird die Erfindung anhand eines Ausführungsbeispiels unter Bezugnahme auf zwei Zeichnungen weiter erläutert. Es zeigen schematisch:
- Fig. 1: die Rückansicht eines bevorzugten Ausführungsbeispiels der Erfindung in perspektivischer Darstellung und
- Fig. 2: eine Objekt-Lagervorrichtung des bevorzugten Ausführungsbeispiels gemäß Fig. 1 in Vorderansicht.

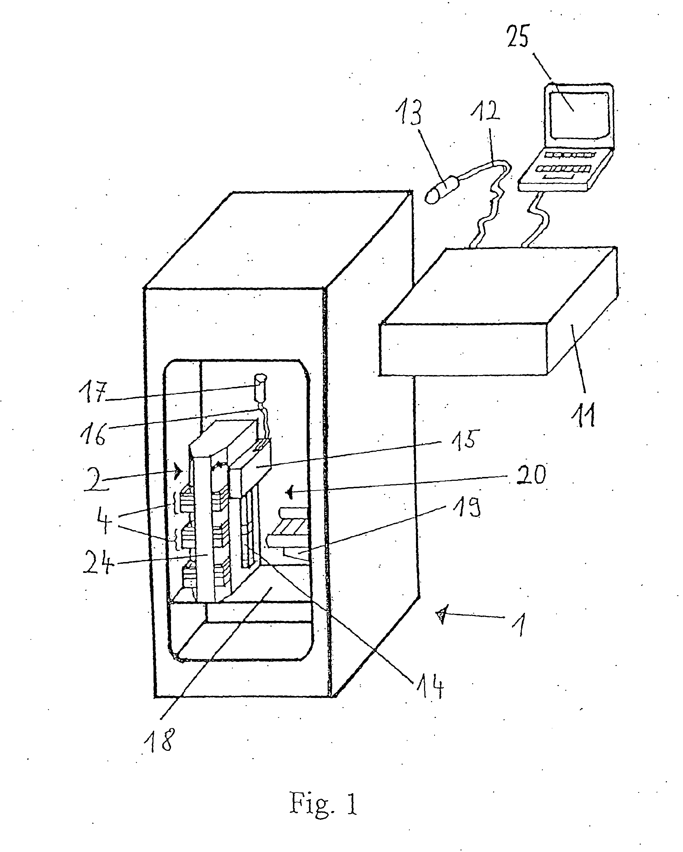

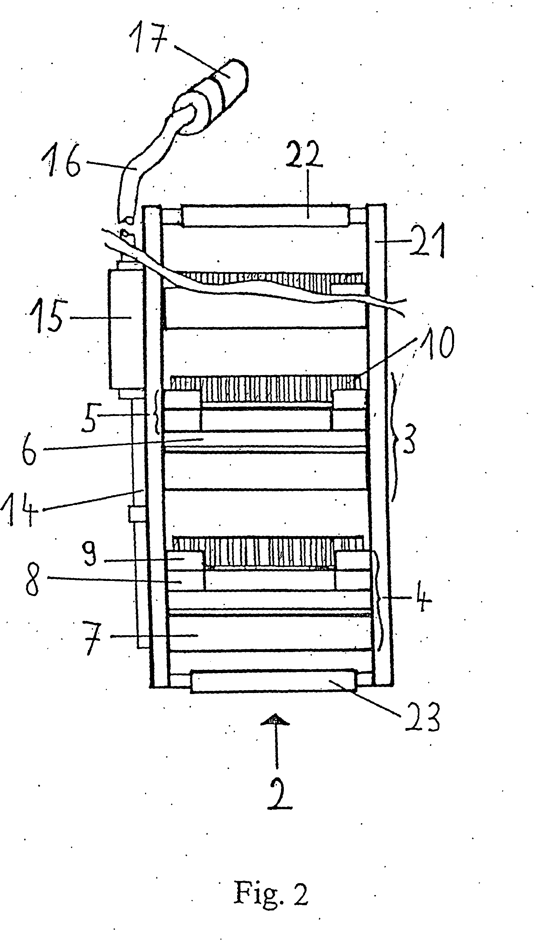

Fig. 1 zeigt einen Schüttel-Inkubator 1, dessen Inkubatorarbeitsraum 20 durch die nicht eingezeichnete Rückwand/Tür einsehbar ist. Im Inkubatorarbeitsraum 20 ist eine Objekt-Lagervorrichtung 2, welche mehrere übereinander angeordnete Objekt-Lagerplätze 3 (siehe Fig. 2) aufweist, dargestellt. An jedem Objekt-Lagerplatz der abgebildeten Objekt-Lagervorrichtung 2 ist eine Schütteleinheit 4 angeordnet. Die Schütteleinheiten 4 sind über Leitungsverbindungen, die sich nicht sichtbar hinter der Abdeckung 14 befinden, mit der Verteilungseinheit 15 verbunden.
Die Verteilungseinheit 15 ist wiederum über die Leitungsverbindung 16 und den Leitungsanschluss 17 lösbar an den Leitungsanschluss 13 der Steuer-/Versorgungsleitung 12 der Steuereinheit 11 angeschlossen. Im vorliegenden Ausführungsbeispiel umfasst die Steuereinheit 11 eine Recheneinheit 25, die der Festlegung der Schüttelbewegung der Schüttelplattform 6 einer sich auf der Objekt-Lagervorrichtung 2 befindlichen Schütteleinheit 4 dient. Die Kommunikation der Recheneinheit 25 mit den übrigen Komponenten der Steuereinheit 11 erfolgt im vorliegenden Beispiel über eine RS232-Schnittstelle. Grundsätzlich sind dem Fachmann zu diesem Zwecke aber auch andere Schnittstellen bekannt.

Der in Fig. 1 abgebildete Schüttel-Inkubator 1 weist im Inkubatorarbeitsraum 20 lediglich eine Objekt-Lagervorrichtung 2 auf, die auf einer Montageplatte 18 angeordnet ist. Grundsätzlich sind dem Fachmann aber auch Anordnungen mehrerer Objekt-Lagervorrichtungen 2 bekannt, darunter auch eine karussellartige Anordnung, wie sie in der WO 98/05753 offenbart ist.
Bei der im Inkubatorarbeitsraum 20 teilweise dargestellten Vorrichtung 19 handelt es sich um ein Transportsystem, wie es aus dem Stand der Technik, zum Beispiel der WO 98/05753, bekannt ist. Mit dem Transportsystem 19 können Objekte 10 zu den einzelnen Objekt-Lagerplätzen 3 einer Objekt-Lagervorrichtung 2 transportiert und dort auf eine Objekt-Lagerstelle 5 gelegt werden (siehe Fig. 2). Entsprechend wird ein Objekt 10 bei Bedarf durch das Transportsystem 19 von der Objekt-Lagerstelle 5 wieder entnommen. Durch die Verwendung eines Transportsystems 19 und die Anordnung mindestens einer erfindungsgemäßen Schütteleinheit 4 an einer Objekt-Lagervorrichtung 2 ist es möglich, die Vorzüge einer automatisierten Be- und Entladung der Objekt-Lagerstellen 5 mit Objekten 10 und einer automatisierten Schüttelung der Objekte 10 in einem einzigen Inkubator miteinander zu verbinden.

Fig. 2 zeigt die Vorderansicht einer Objekt-Lagervorrichtung 2, wobei erfindungsgemäß an mehreren Objekt-Lagerplätzen 3 je eine Schütteleinheit 4 angeordnet ist. Die Objekt-Lagervorrichtung besteht im wesentlichen aus einem turmförmigen Aufbau mit zwei Seitenwänden 21, einer Deckenplatte 22 und einer Bodenplatte 23. Mit Hilfe einer in der Bodenplatte 23 befindlichen Zentrieröffnung (nicht abgebildet) und einer auf der Montageplatte 18 angebrachten Zentrierleiste (nicht abgebildet), die mit der Zentrieröffnung formschlüssig zusammenwirkt, ist die Objekt-Lagervorrichtung 2 sicher und gegen ein etwaiges Verrutschen geschützt auf der Montageplatte 18 positioniert. Darüber hinaus kann die Objekt-Lagervorrichtung 2, wie in Fig. 1 dargestellt eine Rückwand 24 aufweisen, die der Objekt-Lagervorrichtung eine zusätzliche Stabilität verleiht.

Je nach Raumbedarf der zu lagernden Objekte, können in einer Objekt-Lagervorrichtung 2 typischerweise vier bis sechs Schütteleinheiten 4 angeordnet werden. Eine Schütteleinheit 4 umfasst eine Objekt-Lagerstelle 5, eine Schüttelplattform 6 und eine Grundeinheit 7. Wie im vorliegenden Ausführungsbeispiel, kann ein Verrutschen des Objektes 10 durch die Spannelemente 9 wirksam verhindert werden. Diese sind vorliegend als Fixierwinkel auf den vier Ecken der Objekt-Lagerstelle 5 ausgebildet. Wie aus Fig. 2 ersichtlich ist, umfasst die Objekt-Lagerstelle 5 auch ein Distanzelement 8. Dieses ist auf der Schüttelplattform 6 angeordnet und so ausgeformt, dass das Transportsystem 19 ein Objekt 10 von unten her halternd auf eine Objekt-Lagerstelle 5 legen und bei Bedarf wieder entfernen kann. Bei einem anders ausgebildeten Transportsystem, das Objekte 10 beispielsweise von ihrer Oberseite her haltert, kann auf die Distanzelemente 8 verzichtet werden. Wie in Fig. 2 dargestellt, sind Objekt 10, Spannelemente 9, und Distanzelement 8 auf der Schüttelplattform 6 angeordnet. Das heißt, Objekt 10 wird samt der Objekt-Lagerstelle 5 durch die Schüttelplattform 6 bewegt. Die Schüttelbewegung der Schüttelplattform 6 wird durch die Grundeinheit 7, auf der die Schüttelplattform 6 angeordnet ist, herbeigeführt. Die Grundeinheit 7 kann, wie im vorliegenden Ausführungsbeispiel dargestellt, fest mit der Objekt-Lagervorrichtung 2 verbunden sein, wodurch sich für die Objekt-Lagervorrichtung 2 eine zusätzliche Stabilisierung ergibt.

Ein in Fig. 2 dargestelltes Objekte 10 ist zusammen mit Spannelementen 9 und Distanzelement 8 auf der Schüttelplattform 6 angeordnet, die bezüglich der Grundeinheit 7 zentriert und parallel, d. h. horizontal ausgerichtet ist. Dabei erfolgt die Positionierung der Schüttelplattform 6 auf eine zentrische Nullstellung nach (Strom-)Abschaltung selbständig und ohne weiteren Eingriff von außen. Dadurch kommt ein Objekt 10 nach (Strom-)Abschaltung in einer definierten räumlichen Position zu liegen ohne dass eine aufwendige elektrische Nachführung der Schüttelplattform 6 notwendig wäre. Eine exakt bekannte räumliche Position ist erforderlich, damit das automatisierte Transportsystem 19 ein Objekt 10 von einer Objekt-Lagerstelle 5 nehmen bzw. auf eine Objekt-Lagerstelle 5 legen kann.

Die Schütteleinheiten 4 der in Fig. 2 abgebildeten Objekt-Lagervorrichtung 2 sind über Leitungsverbindungen, die sich unter der Abdeckung 14 befinden, an die Verteilungseinheit 15 angeschlossen, die an einer Seitenwand 21 der Objekt-Lagervorrichtung 2 angeordnet ist. Die Verteilungseinheit 15 ist mit dem Leitungsanschluss 17 an den Leitungsanschluss 13 der Steuereinheit 11 angeschlossen. Somit sind im vorliegenden Ausführungsbeispiel letztlich alle Schütteleinheiten 4, die sich auf der abgebildeten Objekt-Lagervorrichtung 2 befinden, über nur einen lösbaren Leitungsanschluss 17 an die der Steuerung und Stromversorgung dienende Steuereinheit 11 angeschlossen.

Wie aus Fig. 2 ersichtlich ist, ist der Abstand zwischen den sich auf der Objekt-Lagervorrichtung 2 befindlichen Schütteleinheiten 4 so gewählt, dass auch höhere, als die abgebildeten Objekte 10, geschüttelt werden können. Konkret bedeutet dies, dass neben den abgebildeten Objekten 10 mit einer Höhe von ca. 25 mm auch Objekte bis zu einer Höhe von ca. 55 mm geschüttelt werden können. Da die Höhe der Schütteleinheiten 4 bekannt ist und im vorliegenden Ausführungsbeispiel ca. 45 mm beträgt, ist es unter Heranziehung der genannten Maßangaben jederzeit möglich, die Anzahl der Schütteleinheiten 4 auf der Objekt-Lagervorrichtung 2 so zu verändern, dass sie den jeweiligen Erfordernissen genügt.

## Patentansprüche

1. Schüttel-Inkubator mit mindestens einer Objekt-Lagervorrichtung, welche mehrere übereinander angeordnete Objekt-Lagerplätze aufweist,
**dadurch gekennzeichnet,**
**dass** an mehreren Objekt-Lagerplätzen (3) je eine Schütteleinheit (4), umfassend eine Objekt-Lagerstelle (5), eine Schüttelplattform (6) und eine Grundeinheit (7), angeordnet ist und jede Schüttelplattform (6) individuell und von den anderen Schüttelplattformen (6) unabhängig von mindestens einer Steuereinheit (11) ansteuerbar ist.

2. Schüttel-Inkubator nach Anspruch 1,
**dadurch gekennzeichnet,**
**dass** die Grundeinheit (7) der Schütteleinheit (4) fest mit der Objekt-Lagervorrichtung (2) verbunden ist.

3. Schüttel-Inkubator nach Anspruch 1,
**dadurch gekennzeichnet,**
**dass** an dem Objekt-Lagerplatz (3) eine lösbare Halterung für die Schütteleinheit (4) ausgebildet ist, so dass die Schütteleinheit (4) bei Bedarf aus der Objekt-Lagervorrichtung (2) entfernt werden kann.

4. Schüttel-Inkubator nach einem der vorhergehenden Ansprüche,
**dadurch gekennzeichnet,**
**dass** die Objekt-Lagerstelle (5) der Schütteleinheit (4) ausgebildet ist, um durch ein automatisiertes Transportsystem (19) mit einem Objekt (10) bestückt zu werden und um mit einem automatisierten Transportsystem (19) ein Objekt (10) von der Objekt-Lagerstelle (5) zu entfernen.

5. Schüttel-Inkubator nach Anspruch 4,
**dadurch gekennzeichnet,**
**dass** die Objekt-Lagerstelle (5) der Schütteleinheit (4) ein auf der Schüttelplattform (6) angeordnetes Distanzelement (8) umfasst, welches Freiraum zur Manipulation eines sich auf der Objekt-Lagerstelle (5) befindlichen Objektes (10) schafft.

6. Schüttel-Inkubator nach einem der vorhergehenden Ansprüche,
**dadurch gekennzeichnet,**
**dass** die Objekt-Lagerstelle (5) der Schütteleinheit (4) mindestens ein auf der Schüttelplattform (6) oder dem Distanzelement (8) angeordnetes Spannelement (9) umfasst.

7. Schüttel-Inkubator nach einem der vorhergehenden Ansprüche,
**dadurch gekennzeichnet,**
**dass** die mindestens eine Steuereinheit (11) zur Steuerung und Stromversorgung der Schütteleinheiten (4) außerhalb eines Inkubatorarbeitsraumes (20) angeordnet ist und von der Steuereinheit (11) aus eine Steuer-/Versorgungsleitung (12) in den Inkubatorarbeitsraum (20) führt, wobei die Steuer-/Versorgungsleitung (12) im Inkubatorarbeitsraum (20) einen Leitungsanschluss (13) aufweist.

8. Schüttel-Inkubator nach Anspruch 7,
**dadurch gekennzeichnet,**
**dass** die Schütteleinheit (4) über eine lösbare Leitungsverbindung an den Leitungsanschluss (13) der mindestens einen Steuereinheit (11) angeschlossen ist.

9. Schüttel-Inkubator nach Anspruch 7 oder 8,
**dadurch gekennzeichnet,**
**dass** im Inkubatorarbeitsraum (20) eine Verteilungseinheit (15) zum Anschluss mehrerer Schütteleinheiten (4) angeordnet ist, wobei die Verteilungseinheit (15) über eine lösbare Leitungsverbindung (16) an den Leitungsanschluss (13) angeschlossen ist.

10. Schüttel-Inkubator nach Anspruch 7 bis 9,
**dadurch gekennzeichnet,**
**dass** an einer Objekt-Lagervorrichtung (2) eine Verteilungseinheit (15) zum Anschluss mehrerer Schütteleinheiten (4) angeordnet ist.

11. Schüttel-Inkubator nach Anspruch 7 bis 10,
**dadurch gekennzeichnet,**
**dass** an mehreren Objekt-Lagervorrichtungen (2) eine Verteilungseinheit (15) zum Anschluss mehrerer Schütteleinheiten (4) angeordnet ist.

12. Schüttel-Inkubator nach einem der vorhergehenden Ansprüche,
**dadurch gekennzeichnet,**
**dass** die Schüttelplattform (6) einer Schütteleinheit (4) ausgebildet ist, sich nach (Strom-)Abschaltung selbständig auf eine zentrische Nullstellung zu positionieren.

## Claims

1. A shaking incubator having at least one object storage device, which has multiple object storage spaces situated one above another,
**characterized in that** one shaking unit (4), comprising an object storage point (5), a shaking platform (6), and a base unit (7), is situated on each of multiple object storage spaces (3), and each shaking platform (6) is activatable individually and independently of the other shaking platforms (6) by at least one control unit (11).

2. The shaking incubator according to Claim 1,
**characterized in that** the base unit (7) of the shaking unit (4) is permanently connected to the object storage device (2).

3. The shaking incubator according to Claim 1,
**characterized in that** a removable retainer for the shaking unit (4) is implemented on the object storage space (3), so that the shaking unit (4) may be removed as needed from the object storage device (2).

4. The shaking incubator according to one of the preceding claims,
**characterized in that** the object storage point (5) of the shaking unit (4) is implemented to be equipped with an object (10) by an automated transport system (19) and to remove an object (10) from the object storage point (5) using an automated transport system (19).

5. The shaking incubator according to Claim 4,
**characterized in that** the object storage point (5) of the shaking unit (4) comprises a spacer element (8) situated on the shaking platform (6), which provides free space for manipulating an object (10) located on the object storage point (5).

6. The shaking incubator according to one of the preceding claims,
**characterized in that** the object storage point (5) of the shaking unit (4) comprises at least one clamping element (9) situated on the shaking platform (6) or the spacer element (8).

7. The shaking incubator according to one of the preceding claims,
**characterized in that** the at least one control unit (11) for control and power supply of the shaking units (4) is situated outside an incubator operating chamber (20) and a control/power line (12) leads from the control unit (11) into the incubator operating chamber (20), the control/power line (12) having a line terminal (13) in the incubator operating chamber (20).

8. The shaking incubator according to Claim 7,
**characterized in that** the shaking unit (4) is connected via a detachable line connection to the line terminal (13) of the at least one control unit (11).

9. The shaking incubator according to Claim 7 or 8,
**characterized in that** a distribution unit (15) for connecting multiple shaking units (4) is situated in the incubator operating chamber (20), the distribution unit (15) being connected via a detachable line connection (16) to the line terminal (13).

10. The shaking incubator according to Claims 7 through 9,
**characterized in that** a distribution unit (15) for connecting multiple shaking units (4) is situated on an object storage device (2).

11. The shaking incubator according to Claims 7 through 10,
**characterized in that** a distribution unit (15) for connecting multiple shaking units (4) is situated on multiple object storage devices (2).

12. The shaking incubator according to one of the preceding claims,
**characterized in that** the shaking platform (6) of a shaking unit (4) is implemented to automatically position itself at a central neutral position after (power) shutdown.

## Revendications

1. Incubateur agitateur avec au moins un dispositif porte-objet possédant plusieurs emplacements d'objet superposés, **caractérisé en ce qu'**il est prévu pour chacun d'une pluralité d'emplacements d'objet (3) une unité d'agitateur (4) comprenant un réceptacle d'objet (5), une plate-forme d'agitation (6) et une unité de base (7), et chaque plate-forme d'agitation (6) peut être actionnée individuellement et indépendamment des autres plates-formes d'agitation (6) par au moins une unité de commande (11).

2. Incubateur agitateur selon la revendication 1, **caractérisé en ce que** l'unité de base (7) de l'unité d'agitateur (4) est assemblée de manière fixe avec le dispositif porte-objet (2).

3. Incubateur agitateur selon la revendication 1, **caractérisé en ce qu'**il est prévu sur l'emplacement d'objet (3) une fixation pouvant être ouverte pour l'unité d'agitateur (4), de sorte que l'unité d'agitateur (4) peut être retirée en cas de besoin du dispositif porte-objet (2).

4. Incubateur agitateur selon l'une des revendications précédentes, **caractérisé en ce que** le réceptacle d'objet (5) de l'unité d'agitateur (4) est conçu pour être garni d'un objet (10) par un système de transport automatique (19) et pour que le système de transport automatique (19) puisse enlever un objet (10) du réceptacle d'objet (5).

5. Incubateur agitateur selon la revendication 4, **caractérisé en ce que** le réceptacle d'objet (5) de l'unité d'agitateur (4) comprend un élément d'écartement (8) disposé sur la plate-forme d'agitation (6), qui crée un espace libre pour la manipulation d'un objet (10) se trouvant sur le réceptacle d'objet (5).

6. Incubateur agitateur selon l'une des revendications précédentes, **caractérisé en ce que** le réceptacle d'objet (5) de l'unité d'agitateur (4) comprend au moins un élément de serrage (9) disposé sur la plate-forme d'agitation (6) ou l'élément d'écartement (8).

7. Incubateur agitateur selon l'une des revendications précédentes, **caractérisé en ce que** l'unité de commande (11) au nombre d'une au moins destinée à la commande et à l'alimentation électrique des unités d'agitateur (4) est disposée à l'extérieur d'un espace de travail de l'incubateur (20) et une ligne de commande et d'alimentation (12) va de l'unité de commande (11) à l'intérieur de l'espace de travail de l'incubateur (20), laquelle ligne de commande et d'alimentation (12) présente dans l'espace de travail de l'incubateur (20) un raccord de ligne (13).

8. Incubateur agitateur selon la revendication 7, **caractérisé en ce que** l'unité d'agitateur (4) est raccordée par une connexion de ligne pouvant être défaite au raccord de ligne (13) de l'unité de commande (11) au nombre d'une au moins.

9. Incubateur agitateur selon la revendication 7 ou 8, **caractérisé en ce qu'**il est prévu dans l'espace de travail de l'incubateur (20) une unité de distribution (15) pour le branchement de plusieurs unités d'agitateur (4), l'unité de distribution (15) étant raccordée au raccord de ligne (13) par une connexion de ligne (16) pouvant être défaite.

10. Incubateur agitateur selon les revendications 7 à 9, **caractérisé en ce qu'**il est prévu sur un dispositif porte-objet (2) une unité de distribution (15) pour le branchement de plusieurs unités d'agitateur (4).

11. Incubateur agitateur selon la revendication 7 à 10, **caractérisé en ce qu'**il est prévu sur plusieurs dispositifs porte-objet (2) une unité de distribution (15) pour le branchement de plusieurs unités d'agitateur (4).

12. Incubateur agitateur selon l'une des revendications précédentes, **caractérisé en ce que** la plate-forme d'agitation (6) d'une unité d'agitateur (4) est conçue pour se positionner automatiquement dans une position neutre centrée après l'arrêt (de l'alimentation électrique).
